# EUROPEAN PATENT APPLICATION

(11) **EP 4 570 323 A1**
(43) Date of publication of application: **18.06.2025**
(21) Application number: 23852370.8
(22) Date of filing: 25.07.2023
(51) Int. Cl.: A61Q 1/02, A61K 8/06, A61K 8/19, A61K 8/37, A61K 8/73, A61K 8/85

(54) **OIL-IN-WATER EMULSION-TYPE COSMETIC COMPOSITION**

(30) Priority: 08.08.2022 JP 2022126300
(71) Applicant: Shiseido Company, Ltd., Chuo-ku Tokyo 104-0061 (JP)
(72) Inventor: SAINO, Ryota, Tokyo 104-0061 (JP); HASEGAWA, Katsuyuki, Tokyo 104-0061 (JP)
(74) Representative: Ter Meer Steinmeister & Partner
(86) International application number: PCT/JP2023/027255
(87) International publication number: WO 2024/034394

(57) **Abstract**

Provided is an oil-in-water emulsified cosmetic composition having a characteristic fresh feel during use while exhibiting excellent emulsified stability and high covering power. The oil-in-water emulsified cosmetic composition comprises the following components (a) to (d): (a) pigment particles with a mean particle size of 200 to 500 nm and hydrophobically treated on the surface, (b) a water-soluble alkyl-substituted polysaccharide derivative, (c) polyhydroxystearic acid, and (d) an oil containing a polar oil, wherein the pigment particles are dispersed in the oil, the content of the pigment particles is 8.0 mass% to 15 mass%, and the content of nonionic surfactants with an HLB value of 10 or greater is 1.0 mass% or lower.

## Description

### FIELD

The present invention relates to an oil-in-water emulsified cosmetic composition.

### BACKGROUND

Oil-in-water emulsified cosmetic compositions that include an aqueous phase as the continuous phase impart a fresh and refreshing feel during use, and are widely used as base materials for makeup cosmetics such as foundations, for makeup primers, and for basic skincare materials such as sunscreens and emulsions.

For example, PTL 1 discloses the following oil-in-water emulsified cosmetic composition: An oil-in-water cosmetic composed of an aqueous phase and an oil phase, and comprising a hydrophobized inorganic powder, a fatty acid soap, a nonionic surfactant and a hydrophilic polymer, wherein the aqueous phase comprises an aqueous medium and the oil phase comprises a non-silicone oil-containing lubricant, the hydrophobized inorganic powder comprises at least a hydrophobized iron oxide powder and a hydrophobized pigment-grade titanium oxide powder, hydrophobized inorganic powders of the same type or different types are present in the oil phase and aqueous phase, the contents of the hydrophobized pigment-grade titanium oxide powder in the oil phase and the aqueous phase are greater than 0.5 mass% and lower than 5 mass%, and greater than 2.5 mass% and lower than 20 mass%, respectively, based on the total amount of the oil-in-water cosmetic, either one type or two or more different types of nonionic surfactant are included, the overall HLB value is 6 to 15, the hydrophilic polymer comprises a hydrophilic polymer that includes as a repeating unit an ethylenically unsaturated compound having an aminoalkylsulfonic acid or its salt as a substituent, and the total content of the non-silicone oil is greater than 5 mass% and lower than 30 mass% based on the total amount of the oil-in-water cosmetic.

### [CITATION LIST]

### [PATENT LITERATURE]

[PTL 1] Japanese Patent Public Inspection No. 2022-516239

### SUMMARY

### [TECHNICAL PROBLEM]

In an oil-in-water emulsified cosmetic such as an oil-in-water foundation it is usually necessary to add large amounts of a pigment particle component having a particle diameter of about 200 to 500 nm, in order to cover either irregularities in the skin or color spots such as blemishes or freckles.

However, large addition (a high content) of a pigment particle component impairs the emulsified stability of the cosmetic, or may result in insufficient dispersibility of the pigment particles.

The present invention provides an improvement to this situation, its object being to provide an oil-in-water emulsified cosmetic composition having a characteristic fresh feel during use while exhibiting excellent emulsified stability and high covering power.

### [SOLUTION TO PROBLEM]

The present invention achieves the object described above by the following aspects.

### <Aspect 1>

An oil-in-water emulsified cosmetic composition comprising the following components (a) to (d):
(a) pigment particles with a mean particle size of 200 to 500 nm and hydrophobically treated on a surface,
(b) a water-soluble alkyl-substituted polysaccharide derivative,
(c) polyhydroxystearic acid, and
(d) an oil containing a polar oil,

wherein the pigment particles are dispersed in the oil,
wherein a content of the pigment particles is 8.0 mass% to 15 mass%, and
wherein a content of nonionic surfactants with an HLB value of 10 or greater is 1.0 mass% or lower.

### <Aspect 2>

The composition according to aspect 1, wherein the content of the polyhydroxystearic acid is 0.1 mass% to 1.5 mass%.

### <Aspect 3>

The composition according to aspect 1 or 2, wherein 50 mass% or greater of the oil is the polar oil.

### <Aspect 4>

The composition according to any one of aspects 1 to 3, wherein the water-soluble alkyl-substituted polysaccharide derivative is hydrophobic-modified hydroxypropyl methylcellulose.

### <Aspect 5>

The composition according to any one of aspects 1 to 4, wherein a content of the water-soluble alkyl-substituted polysaccharide derivative is 0.05 mass% to 1.0 mass%.

### <Aspect 6>

The composition according to any one of aspects 1 to 5, which further comprises an aqueous phase thickener.

### <Aspect 7>

The composition according to any one of aspects 1 to 6, wherein a content of the oil is 5.0 mass% to 40 mass%.

### <Aspect 8>

The composition according to any one of aspects 1 to 7, wherein the polar oil comprises an ultraviolet absorber.

### <Aspect 9>

The composition according to any one of aspects 1 to 8, which is a makeup cosmetic composition.

### <Aspect 10>

The composition according to any one of aspects 1 to 9, which is a foundation.

### [ADVANTAGEOUS EFFECTS OF INVENTION]

According to the invention it is possible to provide an oil-in-water emulsified cosmetic composition having a characteristic fresh feel during use while exhibiting excellent emulsified stability and high covering power.

The phrase "characteristic fresh feel during use", as used herein, means a tactile sensation of immediate reduction in viscosity and break up when the cosmetic composition has been applied to the skin with a finger, with a very fresh sensation simultaneously spreading throughout the skin.

### DESCRIPTION OF EMBODIMENTS

Embodiments of the invention will now be explained in detail. The invention is not limited to the embodiments described below, however, and various modifications may be implemented within the scope of the gist thereof.

### <Oil-in-water emulsified cosmetic composition>

The oil-in-water emulsified cosmetic composition of the invention (hereunder also referred to as "composition of the invention") is an oil-in-water emulsified cosmetic composition comprising the following components (a) to (d):
(a) pigment particles with a mean particle size of 200 to 500 nm and hydrophobically treated on the surface,
(b) a water-soluble alkyl-substituted polysaccharide derivative,
(c) polyhydroxystearic acid, and
(d) an oil containing a polar oil,
wherein:
the pigment particles are dispersed in the oil,
the content of the pigment particles is 8.0 mass% to 15 mass%, and
the content of nonionic surfactants with an HLB value of 10 or greater is 1.0 mass% or lower.

The composition of the invention has a characteristic fresh feel during use while exhibiting excellent emulsified stability and high covering power. Without being limited to any particular theory, this is believed to be due to the characteristic construction of the composition of the invention.

More specifically, the composition of the invention has high covering power because it comprises component (a) (pigment particles having a mean particle size of 200 to 500 nm and being hydrophobically treated on the surface) in an amount of 8.0 mass% or greater. The composition of the invention also includes component (c) (polyhydroxystearic acid) together with component (a) mentioned above, thereby improving the dispersibility of component (a) in the oil. The composition of the invention further includes component (b) (a water-soluble alkyl-substituted polysaccharide derivative), with the content of nonionic surfactants with an HLB value of 10 or greater being limited to 1.0 mass% or lower, whereby it exhibits a characteristic fresh feel during use. The water-soluble alkyl-substituted polysaccharide derivative has relatively weak stabilizing action for emulsified particles, compared to one with a nonionic surfactant having an HLB value of 10 or greater. However, because emulsified particles obtained by the water-soluble alkyl-substituted polysaccharide derivative have relatively large particle sizes, it is possible to include a relatively large amount of component (a) in the emulsified particles, allowing the water-soluble alkyl-substituted polysaccharide derivative to improve the emulsified stability of the composition of the invention.

More specifically, the content of nonionic surfactants with an HLB value of 10 or greater in the composition of the invention may be 1.0 mass% or lower, 0.9 mass% or lower, 0.8 mass% or lower, 0.7 mass% or lower, 0.6 mass% or lower, 0.5 mass% or lower, 0.4 mass% or lower, 0.3 mass% or lower, 0.2 mass% or lower, 0.1 mass% or lower, or 0 mass% (i.e. substantially absent).

Without being restrictive, the composition of the invention may have an emulsified particle size with an average of 1 to 5 µm and preferably an average of 1 to 10 µm, and a maximum of about 20 µm. According to the invention, the emulsified particle size can be determined, for example, by using a microscope for overall visual assessment of the sizes of the emulsified particles (measured with a micrometer) and their distribution (the uniformity of particles in the specular field of view).

### <(a) Pigment particles>

The pigment particles in the composition of the invention have a mean particle size of 200 to 500 nm, and the pigment particles are hydrophobically treated on the surface.

Since the mean particle size of the pigment particles is 200 to 500 nm, they are able to cover irregularities in the skin and color spots such as blemishes and freckles, especially against visible light.

More specifically, the mean particle size of the pigment particles of the invention may be 200 nm or larger, 250 nm or larger or 300 nm or larger, and 500 nm or smaller, 450 nm or smaller, 400 nm or smaller or 350 nm or smaller, for example.

The "mean particle size" referred to herein is the mean particle size of the primary particles. The mean particle size can be calculated as the projected circle equivalent diameter of the primary particles as observed by SEM or TEM.

The type of pigment particles used for the invention is not particularly restricted and may be surface hydrophobic-treated iron oxide or titanium oxide, for example. Iron oxide used in the pigment particles of the invention may be yellow iron oxide, red iron oxide or black iron oxide, or iron oxides of different colors may be combined for adjustment to the desired composition color.

According to the invention there is no particular restriction on the type of hydrophobic treatment agent for surface hydrophobic treatment of the pigment particles, and examples include fatty acids, higher fatty acids, higher alcohols, hydrocarbons, triglycerides, esters, silicone oils, silicone resins and fluorine compounds. More specific examples of surface hydrophobic treatment agents to be used for iron oxide or titanium oxide include, but are not limited to, silicones (especially alkyl-modified silicones), alkyltriethoxysilanes, alkyltrimethoxysilanes, perfluoroalkyl phosphates, (alkyl acrylate/dimethylsilicone) copolymers, dextrin palmitate, triethoxysilylethyl polydimethylsiloxyethylhexyldimethicone, monomethylpolysiloxane, dimethylpolysiloxane, polymer silicones, and sodium acryloyldimethyltaurate/methacrylamidelaurylic acid copolymer. From the viewpoint of improving the powder dispersibility, iron oxide is preferably subjected to silicone treatment and especially alkyl-modified silicone treatment, and titanium oxide is preferably subjected to silicone treatment or alkyltrimethoxysilane treatment.

The shapes of the pigment particles of the invention are not particularly restricted and may be spherical, laminar, rod-shaped, fusiform, needle-like or amorphous, and the like.

The content of the pigment particles in the composition of the invention with respect to the total composition may be 8.0 mass% or greater, 9.0 mass% or greater, 10 mass% or greater, 11 mass% or greater, 12 mass% or greater, 13 mass% or greater or 14 mass% or greater, for example, from the viewpoint of improving the covering power of the composition, and 15 mass% or lower, 14 mass% or lower, 13 mass% or lower, 12 mass% or lower or 11 mass% or lower, for example, from the viewpoint of improving the emulsified stability and dispersibility of the pigment particles.

The pigment particles in the composition of the invention are dispersed in the oil. Such a state allows the effect of the invention to be exhibited even without re-dispersion by a subsequent procedure such as shaking when the composition of the invention is applied to the skin.

### <(b) Water-soluble alkyl-substituted polysaccharide derivative>

The water-soluble alkyl-substituted polysaccharide derivative in the composition of the invention is not particularly restricted, and may be a hydrophobic-modified alkyl cellulose or a hydrophobic-modified sulfonated polysaccharide derivative. A water-soluble alkyl-substituted polysaccharide derivative, and particularly a hydrophobic-modified alkyl cellulose, for the purpose of the invention, is one that does not qualify as a "nonionic surfactant having an HLB value of 10 or greater", and that is generally classified as a "polymer surfactant".

### (Hydrophobic-modified alkyl cellulose)

A "hydrophobic-modified alkyl cellulose", for the purpose of the invention, is an alkyl cellulose hydrophobically modified by an alkyl group of 14 to 22 carbon atoms. The hydrophobic-modified alkyl cellulose is a compound having a long chain alkyl group as a hydrophobic group introduced into a water-soluble cellulose ether derivative, and it is represented by the following general formula (I).

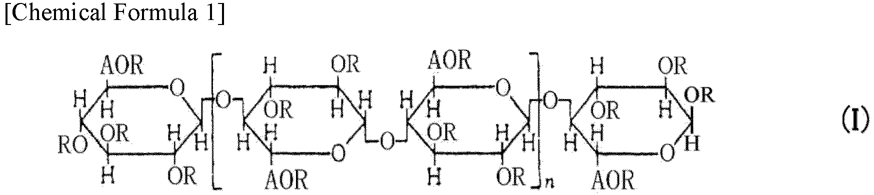

[In the formula, each R may be the same or different and represents one or more groups selected from among a hydrogen atom, alkyl groups of 1 to 4 carbon atoms, the group - [CH₂CH(CH₃)O]ₘ-H (where m is an integer of 1 to 5 and preferably 1 to 3), the group - CH₂CH₂OH and the group -CH₂CH(OH)CH₂OR' (where R' is an alkyl group of 14 to 22 carbon atoms), with the proviso that the group -CH₂CH(OH)CH₂OR' is definitely included. Also in the formula, "A" represents the group -(CH₂)_{q}- (where q is an integer of 1 to 3 and preferably 1), and n represents an integer of 100 to 10,000 and preferably 500 to 5000.]

The method for producing a hydrophobic-modified alkyl cellulose of formula (I) may be contacting a water-soluble cellulose ether derivative, and specifically methyl cellulose (where R is a hydrogen atom or methyl group), ethyl cellulose (where R is a hydrogen atom or an ethyl group), propyl cellulose (where R is a hydrogen atom or a propyl group), butyl cellulose (where R is a hydrogen atom or a butyl group), hydroxypropyl cellulose [where R is a hydrogen atom or a hydroxypropyl group (the group -[CH₂CH(CH₃)O]ₘ-H (where m is an integer of 1 to 5 and preferably 1 to 3))] or hydroxypropylmethyl cellulose (where R is a hydrogen atom or a methyl or hydroxypropyl group (same as above)), with a compound serving for introduction of a long chain alkyl group of 14 to 22 carbon atoms, and specifically a long-chain alkylglycidyl ether of the following formula (II), in the presence of an alkali catalyst. 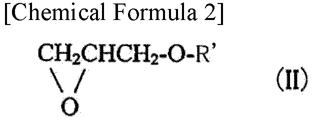 [In the formula, R' represents an alkyl group of 14 to 22 carbon atoms.]

The content of the group -CH₂CH(OH)CH₂OR' to be introduced into the hydrophobic-modified alkyl cellulose of the invention is preferably about 0.1 to 5.0 mass% with respect to the total hydrophobic-modified alkyl cellulose. In order to obtain such a content, production may be carried out with appropriate selection of the molar ratio during reaction between the water-soluble cellulose ester derivative and long-chain alkylglycidyl ether, and the reaction time and type of alkali catalyst used. After the reaction, the reaction product may also be purified by purification steps such as neutralization, filtration, rinsing, drying and sieving.

Hydroxypropyl methylcellulose is most preferably selected among the aforementioned water-soluble cellulose ether derivatives (where R in formula (I) is one of the following four groups: a hydrogen atom, a methyl group, the group -[CH₂CH(CH₃)O]ₘH or the group - CH₂CH(OH)CH₂OR', q for group A is 1, and A is a methylene group).

R' of the long-chain alkylglycidyl ether of formula (II) is an alkyl group of 14 to 22 carbon atoms, preferably an alkyl group of 14 to 20 carbon atoms and even more preferably a stearyl group of 18 carbon atoms (-C₁₈H₃₇). If the number of carbon atoms of the alkyl group R' is less than 14 or 23 or more, the emulsified stability provided by the hydrophobic-modified alkyl cellulose will be insufficient.

The weight-average molecular weight of the hydrophobic-modified alkyl cellulose is preferably 100,000 to 1,000,000, more preferably 300,000 to 800,000, and even more preferably 550,000 to 750,000.

According to the invention, the hydrophobic-modified alkyl cellulose used is preferably hydrophobic-modified hydroxypropyl methylcellulose (hydrophobized hydroxypropyl methylcellulose), and more specifically stearoxyhydroxypropyl methylcellulose, stearoxyhydroxypropyl cellulose or Laureth-13PG hydroxyethyl cellulose. The hydrophobic-modified alkyl cellulose used may be a commercial product, examples of which include but are not limited to SANGELOSE 90L (hydrophobized hydroxypropyl methylcellulose; product of Daido Chemical Corp.), Natrosol Plus 330cs (product of Ashland Co.) and Polysurf 67cs (product of Ashland Co.).

### (Hydrophobic-modified sulfonated polysaccharide derivative)

The hydrophobic-modified sulfonated polysaccharide derivative used for the invention is preferably one with a polysaccharide or its derivative as the basic skeleton, and with all or some of the hydrogen atoms of the hydroxyl groups replaced with the following substituent (a) and/or substituent (b). Substituent (a) is preferably a glyceryl ether group having a hydrophobic portion selected from among alkylglyceryl ether groups with a straight-chain or branched alkyl group of 10 to 40 carbon atoms and alkenylglyceryl ether groups with a straight-chain or branched alkenyl group of 10 to 40 carbon atoms. Substituent (b) is preferably a sulfoalkyl group of 1 to 5 carbon atoms or its salt, preferably substituted with a hydroxyl group. The term "water-soluble" used here means solubility of 0.001 mass% or greater in water at 25°C.

Specific examples for substituent (a) are not particularly restricted and include 2-hydroxy-3-alkoxypropyl, 2-alkoxy-1-(hydroxymethyl)ethyl, 2-hydroxy-3-alkenyloxypropyl and 2-alkenyloxy-1-(hydroxymethyl)ethyl groups. An alkyl group or alkenyl group of 10 to 40 carbon atoms substituting in the glyceryl ether groups is preferably a straight-chain or branched alkyl or alkenyl group of 12 to 36 carbon atoms, or 16 to 24 carbon atoms, among which alkyl groups are preferred, and straight-chain alkyl groups are more preferred.

Specific examples for substituent (b) are not particularly restricted and include 2-sulfoethyl, 3-sulfopropyl, 3-sulfo-2-hydroxypropyl and 2-sulfo-1-(hydroxymethyl)ethyl, all or some of which may form salts with alkali metals such as Na or K, alkaline earth metals such as Ca or Mg, organic cationic groups such as an amines, or ammonium ions.

The degree of substitution of substituent (a) is preferably 0.001 to 1, more preferably 0.002 to 0.5 and even more preferably 0.003 to 0.1, per constituent monosaccharide residue. The degree of substitution of substituent (b) is preferably 0.01 to 2.5, more preferably 0.02 to 2 and even more preferably 0.1 to 1.5, per constituent monosaccharide residue. The proportion of the numbers of substituent (a) and substituent (b) is preferably 1:1,000 to 100:1, more preferably 1:500 to 10:1 and even more preferably 1:300 to 10:1.

The polysaccharide or its derivative serving as the basic skeleton of the hydrophobic-modified sulfonated polysaccharide derivative is not particularly restricted, and it may be cellulose, hydroxyethyl cellulose, methyl cellulose, ethyl cellulose or hydroxypropyl cellulose. The weight-average molecular weight of the polysaccharide or its derivative is preferably 10,000 to 10,000,000, more preferably 100,000 to 5,000,000 and even more preferably 300,000 to 2,000,000.

A specific example for the hydrophobic-modified sulfonated polysaccharide derivative of the invention is stearoxy PG hydroxyethyl cellulose sulfonate, but this is not limitative. The hydrophobic-modified sulfonated polysaccharide derivative used may be a commercial product, such as Poise 310 (stearoxy PG hydroxyethyl cellulose sulfonate by Kao Corp.)

The content of the water-soluble alkyl-substituted polysaccharide derivative in the composition of the invention is not particularly restricted, and for example, it may be 0.05 mass% or greater, 0.10 mass% or greater, 0.15 mass% or greater, 0.20 mass% or greater, 0.25 mass% or greater, 0.30 mass% or greater, 0.35 mass% or greater, 0.40 mass% or greater, 0.45 mass% or greater or 0.50 mass% or greater, and 1.0 mass% or lower, 0.80 mass% or lower, 0.50 mass% or lower or 0.30 mass% or lower, with respect to the total composition.

### <(c) Polyhydroxystearic acid>

The polyhydroxystearic acid in the composition of the invention has an effect of causing the pigment particles to uniformly disperse in the oil.

Polyhydroxystearic acid is an oligomer compound in which hydroxystearic acid forms an ester bond, with examples of commercially available products that may be used including HS Oligomer 600 (by Hokoku Corp.) and SALACOS HS-6C (by Nisshin OilliO Group, Ltd.). The polymerization degree of the polyhydroxystearic acid is not particularly restricted and may be 4 to 8, for example.

The content of the polyhydroxystearic acid in the composition of the invention with respect to the total composition may be 0.1 mass% or greater, 0.2 mass% or greater, 0.3 mass% or greater, 0.4 mass% or greater, 0.5 mass% or greater, 0.6 mass% or greater, 0.7 mass% or greater, 0.8 mass% or greater, 0.9 mass% or greater or 1.0 mass% or greater, for example, from the viewpoint of improving the dispersibility of the pigment particles in the oil, and 1.5 mass% or lower, 1.4 mass% or lower, 1.3 mass% or lower, 1.2 mass% or lower, 1.1 mass% or lower or 1.0 mass% or lower, for example, from the viewpoint of improving the emulsified stability of the pigment particles.

### <(d) Oil>

The oil in the composition of the invention may be one or more selected from among oil components commonly used in cosmetics.

The content of the oil in the composition of the invention is not particularly restricted, but it may be 5.0 mass% or greater, 6.0 mass% or greater, 7.0 mass% or greater, 8.0 mass% or greater, 9.0 mass% or greater, 10.0 mass% or greater, 11.0 mass% or greater, 12.0 mass% or greater, 13.0 mass% or greater, 14.0 mass% or greater, 15.0 mass% or greater or 16.0 mass% or greater, and 40.0 mass% or lower, 30.0 mass% or lower or 20.0 mass% or lower, for example.

The oil used for the invention most preferably includes a polar oil. The emulsified stability of the composition of the invention can be further improved if a polar oil constitutes 40 mass% or greater, 44 mass% or greater, preferably 50 mass% or greater, more preferably 80 mass% or greater, even more preferably 85 mass% or greater, even yet more preferably 90 mass% or greater and most preferably 95 mass% or greater, of the total oil. There is no particular restriction on the upper limit for the percentage of polar oil in the total oil, and for example, 100 mass% of the total oil may be a polar oil, and more specifically, a polar oil may make up 100 mass% or less, 99 mass% or less or 95 mass% or less of the total oil. The composition of the invention may also include a larger amount of polar oil than a conventional cosmetic material, allowing, for example, a high polarity perfume to be stably added.

The content of the polar oil in the composition of the invention is not particularly restricted, and for example, it may be 5.0 mass% or greater, 6.0 mass% or greater, 7.0 mass% or greater, 8.0 mass% or greater, 9.0 mass% or greater, 10.0 mass% or greater, 11.0 mass% or greater or 12.0 mass% or greater, and 40.0 mass% or lower, 30.0 mass% or lower, 20.0 mass% or lower or 15.0 mass% or lower, with respect to the total composition.

The "polar oil" used for the invention is not particularly restricted so long as it has high polarity in oils commonly used in cosmetics, and preferred are oils having a relative permittivity of about 5 or greater, and more preferably about 10 or greater.

Typical examples of polar oils include ester oils, ultraviolet absorbers and polyhydric alcohol polymers.

### (Ester oil)

Specific examples of ester oils that may be used as polar oils for the invention include, but are not limited to, tripropyleneglycol dineopentanoate, isononyl isononanoate, isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, cetyl ethylhexanoate, ethyleneglycol di-2-ethylhexanoate, dipentaerythritol fatty acid esters, N-alkylglycol monoisostearates, neopentylglycol dicaprate, diisostearyl malate, glycerin di-2-heptylundecanoate, trimethylolpropane tri-2-ethylhexanoate, trimethylolpropane triisostearate, pentaerythrityl tetra-2-ethylhexanoate, glycerin triethylhexanoin(tri-2-ethylhexanoate), glycerin trioctanoate, glycerin triisopalmitate, trimethylolpropane triisostearate, cetyl 2-ethyl hexanoate, 2-ethylhexyl palmitate, glycerin trimyristate, tri-2-heptylundecanoic acid glyceride, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate and triethyl citrate.

### (Ultraviolet absorber)

Specific examples of ultraviolet absorbers that may be used as polar oils for the invention include, but are not limited to, benzoic acid derivatives, salicylic acid derivatives, cinnamic acid derivatives, dibenzoylmethane derivatives, β,β-diphenyl acrylate derivatives, benzophenone derivatives, benzylidene camphor derivatives, phenylbenzimidazole derivatives, triazine derivatives, phenylbenzotriazole derivatives, anthranil derivatives, imidazoline derivatives, benzal malonate derivatives and 4,4-diarylbutadiene derivatives. The use of some of these specific compounds and products will be described below.

Examples of benzoic acid derivatives include, but are not limited to, para-aminobenzoic acid (PABA) ethyl, ethyl-dihydroxypropyl PABA, ethylhexyl-dimethyl PABA (such as ESCALOL 507 by ISP Co.), glyceryl PABA, PEG-25-PABA (such as UVINUL P25 by BASF Corp.) and hexyl diethylaminohydroxybenzoylbenzoate (such as UVINUL A PLUS).

Salicylic acid derivatives include, but are not limited to, homosalates (such as EUSOLEX HMS by Rona/EM Industries), ethylhexyl salicylate (such as NeoHeliopan OS by Herman & Reimer), dipropyleneglycol salicylate (such as DIPSAL by Scher Co.) and TEA salicylate (such as NeoHeliopan TS by Herman & Reimer).

Cinnamic acid derivatives include, but are not limited to, ethylhexyl methoxycinnamate (such as PARSOL MCX by Hoffmann-La Roche), isopropyl methoxycinnamate, isoamyl methoxycinnamate (such as NeoHeliopan E1000 by Herman & Reimer), cinnoxate acid, DEA-methoxycinnamate, diisopropyl methylcinnamate, glyceryl-ethyl hexanoate-dimethoxycinnamate and di-(2-ethylhexyl)-4'-methoxybenzalmalonate.

Dibenzoylmethane derivatives include, but are not limited to, 4-tert-butyl-4'-methoxydibenzoylmethane (such as PARSOL 1789).

β,β-Diphenyl acrylate derivatives include, but are not limited to, octocrylene (such as UVINUL N539 by BASF Corp.).

Benzophenone derivatives include, but are not limited to, benzophenone-1(such as UVINUL 400 by BASF Corp.), benzophenone-2 (such as UVINUL D50 by BASF Corp., benzophenone-3 or oxybenzone (such as UVINUL M40 by BASF Corp.), benzophenone-4 (such as UVINUL MS40 by BASF Corp.), benzophenone-5, benzophenone-6 (such as HELISORB 11 by Norquay), benzophenone-8 (such as SPECTRA-SORB UV-24 by American Cyanamide), benzophenone-9 (such as UVINUL DS-49 by BASF Corp.) and benzophenone-12.

Benzylidene camphor derivatives include, but are not limited to, 3-benzylidene camphor (such as MEXORYL SD by Chimex), 4-methylbenzylidene camphor, benzylidene camphor sulfonic acid (such as MEXORYL SL by Chimex), camphor benzalkonium methosulfate (such as MEXORYL SO by Chimex), terephthalylidene dicamphor sulfonic acid (such as MEXORYL SX by Chimex) and polyacrylamidemethylbenzylidene camphor (such as MEXORYL SW by Chimex).

Phenylbenzimidazole derivatives include, but are not limited to, phenylbenzimidazolesulfonic acid (such as EUSOLEX 232 by Merck, Ltd.) and disodium phenyldibenzimidazoletetrasulfonate (such as NeoHeliopan AP by Herman & Reimer).

Triazine derivatives include, but are not limited to, anisotriazine (such as TINOSORB S by Ciba Specialty Chemicals, Inc.), ethylhexyltriazone (such as UVINUL T150 by BASF Corp.), diethylhexyl butamidotriazone (such as UVASORB HEB by Sigma 3V) and 2,4,6-tris(diisobutyl-4'-aminobenzal malonato)-s-triazine.

Phenylbenzotriazole derivatives include, but are not limited to, drometrizole trisiloxane (such as SILATRIZOLE by Rhodia Chimie) and methylenebis(benzotriazolyltetramethylbutylphenol) (such as TINOSORB M by Ciba Specialty Chemicals, Inc.).

Anthranil derivatives include, but are not limited to, menthyl anthranilate (such as NeoHeliopan MA by Herman & Reimer).

Imidazoline derivatives include, but are not limited to, ethylhexyldimethoxybenzylidene dioxoimidazolinepropionate.

Benzal malonate derivatives include, but are not limited to, polyorganosiloxanes with benzal malonate functional groups (such as polysilicone-15; PARSOL SLX by DSM Nutrition, Japan).

4,4-Diarylbutadiene derivatives include, but are not limited to, 1,1-dicarboxy(2,2'-dimethylpropyl)-4,4-diphenylbutadiene.

### (Polyhydric alcohol polymer)

Examples of polyhydric alcohol polymers to be used as polar oils for the composition of the invention include, but are not limited to, diethylene glycol, dipropylene glycol, triethylene glycol, polypropylene glycol, tetraethylene glycol, diglycerin, polyethylene glycol, triglycerin, tetraglycerin and polyglycerin. Polypropylene glycol is more preferred among these from the viewpoint of compatibility.

### (Oils other than polar oils)

The oil used in the composition of the invention may also have an oil other than a polar oil. Oils other than polar oils are not particularly limited, and examples include but are not limited to hydrocarbon oils and silicone oils.

Specific examples of hydrocarbon oils include, but are not limited to, decane, dodecane, isododecane, isohexadecane, undecane, tridecane, liquid paraffin, olefin oligomers, squalane, squalene, paraffins, and mixtures of two or more of the foregoing.

Specific examples of silicone oils include, but are not limited to, straight-chain silicone oils such as polydimethylsiloxane (dimethicone), methylphenylpolysiloxane and methylhydrogen polysiloxane, and cyclic silicone oils such as octamethylcyclotetrasiloxane, decamethylcyclopentasiloxane and dodecamethylcyclohexasiloxane, as well as mixtures of two or more of the foregoing.

When an oil other than a polar oil is included in the composition of the invention, its content (the total content, if two or more oils other than polar oils are included) is not particularly restricted and may be, for example, 0.01 mass% or greater, 0.05 mass% or greater, 0.1 mass% or greater, 0.5 mass% or greater, 1.0 mass% or greater, 2.0 mass% or greater, 3.0 mass% or greater, 4.0 mass% or greater or 5.0 mass% or greater, and 15.0 mass% or lower, 12.0 mass% or lower, 10.0 mass% or lower or 8.0 mass% or lower, with respect to the total composition.

### <Other components>

The composition of the invention may further comprise other components in addition to the essential components (a) to (d), in ranges that do not impair the effect of the invention. Examples of other components are mentioned below.

### (Aqueous phase thickener)

The composition of the invention may further include an aqueous phase thickener. The aqueous phase thickener may be a thickener having the function of thickening the aqueous phase, and in particular, it may be one that reduces the viscosity due to the presence of an electrolyte at a concentration in a range commonly added to cosmetic materials. For the purpose of the invention, an aqueous phase thickener that produces a reduction in viscosity by increasing the electrolyte concentration in this manner will be referred to as "low-salt-resistance aqueous phase thickener". Such a low-salt-resistance thickener is selected from among water-soluble thickeners commonly added to cosmetic materials for the purpose of adjusting the viscosity of the aqueous phase.

Specific examples of aqueous phase thickeners include, but are not limited to, vinyl-based polymers such as polyvinyl alcohol, polyvinyl acetate, polyvinylmethyl ether, polyvinylpyrrolidone, vinylpyrrolidone and vinyl acetate copolymers and carboxyvinyl polymers; and acrylic polymers such as sodium polyacrylate, polyethyl acrylate, alkanolamine polyacrylate, alkyl methacrylate and dimethylaminoethyl methacrylate copolymers, poly 2-acrylamide-2-methylpropanesulfonic acid, polymethacryloyloxytrimethylammonium, (acryloyldimethyltaurineammonium/VP) copolymer and dimethylacrylamide/acryloyldimethyltaurine Na cross polymer.

Preferred among these low-salt-resistance thickeners are thickeners of the type that increase viscosity by formation of a microgel with a water-swelling property in the aqueous phase and friction between swelled microgel particles, compared to thickeners of the type that increase viscosity by entanglement of the polymer chains, because they can help further inhibit a slimy feel when the cosmetic is applied to the skin. This is because hydrophobic-modified alkyl cellulose added as an emulsifying agent has a thickening effect by entanglement of polymer chains, and therefore further addition of a thickener of the type that increases viscosity by the same mechanism can result in a slimy feel due to exacerbation of the thickening effect.

The content of an aqueous phase thickener in the composition of the invention is not particularly restricted, and for example, it may be 0.05 mass% or greater, 0.10 mass% or greater, 0.15 mass% or greater, 0.20 mass% or greater or 0.25 mass% or greater, and 3.0 mass% or lower, 2.0 mass% or lower, 1.0 mass% or lower or 0.5 mass% or lower.

### (Humectant)

The composition of the invention may further include a humectant. Humectants are not particularly restricted, and examples include polyhydric alcohols such as glycerin, 1,3-butylene glycol, dipropylene glycol and propylene glycol; water-soluble polymers such as trehalose, hyaluronic acid and chondroitin sulfate; and tocopherol.

The content of a humectant in the composition of the invention is not particularly restricted, and for example, it may be 0.5 mass% to 15 mass% or 2.0 mass% to 12 mass%.

### (Powder components other than pigment particles)

The composition of the invention may further include a powder component other than the pigment particles. Examples of such powder components include, but are not limited to, extender pigments such as barium sulfate, talc, mica, kaolin, silica, corn starch and cellulose, and polymer powders such as polyethylene powder, poly(methyl methacrylate) powder and nylon powder.

The content of a powder component in the composition of the invention is not particularly restricted, and for example, it may be 0.1 mass% to 10 mass% or 0.1 mass% to 3.0 mass%.

### (Ionic surfactant)

The composition of the invention may further include an ionic surfactant. An ionic surfactant is preferred from the viewpoint of producing electrostatic repulsion to prevent formation of masses by the emulsified particles.

Ionic surfactants are classified as surfactants that dissociate into ions when dissolved in water, and specifically they include anionic surfactants, cationic surfactants and amphoteric surfactants.

Examples of ionic surfactants include, but are not limited to, anionic surfactants such as N-stearoyl-N-methyltaurine, N-stearoyl-N-methyltaurine sodium, disodium N-stearoyl-L-glutamate, sodium N-stearoyl-L-glutamate, sodium polyoxyethylenelauryl ether acetate, triethanolamine polyoxyethylenelauryl ether acetate, sodium taurine laurate, lauroylmethyltaurine sodium and triethanolamine laurate; cationic surfactants such as stearyldimethylamine oxide and stearyltrimethylammonium chloride; and amphoteric surfactants such as lauryldimethyl betaine and betaine lauryldimethylaminoacetate.

The content of an ionic surfactant including an ionic surfactant in the composition of the invention may be, for example, 0.01 to 2.0 mass%, 0.05 to 1.0 mass% or 0.10 to 0.50 mass%.

### (Other surfactants)

For nonionic surfactants, the composition of the invention may also include a nonionic surfactant with an HLB value of less than 10, so long as the content of nonionic surfactants with an HLB value of 10 or greater is lower than 1.0 mass%.

Examples of nonionic surfactants having an HLB value of less than 10 include, but are not limited to, bisbutyldimethicone polyglyceryl-3 (HLB value: 0.5), PEG-10 dimethicone (HLB value: 2.0) and PEG-9 polydimethylsiloxyethyldimethicone (HLB value: 4.0).

The content of such a further included nonionic surfactant with an HLB value of less than 10 in the composition of the invention may be, for example, 0.01 to 2.0 mass% or 0.05 to 1.0 mass%.

A polymer surfactant or natural surfactant may also be added to the composition of the invention in addition to the surfactants mentioned above, in ranges that do not interfere with the effect of the invention.

The composition of the invention may also contain added antioxidants, alcohols, beauty components, lecithin, glycolipids, plant extracts, antiseptic agents, aromatics, pH adjustors or pigments, for example, within ranges that do not interfere with the effect of the invention.

### <Production method and use>

The composition of the invention can be prepared by a method commonly used for oil-in-water emulsified cosmetics. Specifically, the aqueous phase component and oil phase component may be separately mixed, adding the oil phase component while stirring the aqueous phase component, to produce an emulsified mixture.

The composition of the invention has a characteristic fresh feel during use while exhibiting excellent emulsified stability and providing high covering power, and may therefore be used as a makeup cosmetic composition, and especially as a foundation.

### EXAMPLES

The invention will now be further described in greater detail by Examples, with the understanding that the invention is not limited thereto. Unless otherwise specified, the contents are mass% values.

### <Examples and Comparative Examples>

Oil-in-water emulsified cosmetic compositions for the Examples and Comparative Examples were prepared with the formulations listed in Table 1 and Table 2, and the emulsified particle size, feel during use, covering power and emulsified stability of each composition was evaluated based the following criteria, with the results shown in Table 1 and Table 2.

### <Emulsified particle size>

The emulsified particle size for each composition obtained in the Examples and Comparative Examples was determined by using a microscope for overall visual assessment of the sizes of the emulsified particles (measured with a micrometer) and their distribution (the uniformity of particles in the field of view). In Table 1 the expression "1 to 10(20)" means that the diameters of most of the emulsified particles are in the range of about 1 to 10 µm, with a small number of emulsified particles present up to about 20 µm. The same applies for similar expressions.

### <Feel during use>

Each of the compositions obtained in the Examples and Comparative Examples were subjected to overall evaluation for feel during use by a panel of three experts, according to the following scale.
"A": Tactile sensation of rapid break up by reduced viscosity upon application of the cosmetic to skin with a finger, with a very fresh sensation simultaneously spreading throughout the skin;
"B": Slight tactile sensation of rapid break up by reduced viscosity upon application of the cosmetic to skin with a finger, with freshness simultaneously spreading throughout the skin;
"C": No tactile sensation of rapid break up by reduced viscosity upon application of the cosmetic to skin with a finger, and a poor fresh sensation spreading through the skin.

### <Covering power>

Each of the compositions obtained in the Examples and Comparative Examples were subjected to overall evaluation for covering power by a panel of ten experts, according to the following scale.
"A": at least 7 of 10 evaluators reported high covering power;
"B": 4 to 6 of 10 evaluators reported high covering power;
"C" 3 or fewer evaluators reported high covering power.

### <Emulsified stability>

Each of the compositions of the Examples and Comparative Examples was evaluated in a rolling test. More specifically, the rolling test was carried out by filling a cylindrical container halfway with the prepared oil-in-water emulsified cosmetic composition sample, rotating the sample with a rolling tester (product of Nigorikawa Rika Kogyo Co., Ltd.) for 4 hours at 45 rpm, room temperature, and observing any change in state of the composition. Following observation, evaluation was made on the following scale:
"A": Absolutely no noticeable change compared to immediately after preparation;
"B": Faint colored stripes visible compared to immediately after preparation, but not problematic for use;
"C" Oil layer and aqueous layer separated, creating a hindrance for use.

**[Table 1]**

| Table 1 (Examples 1-5, Comparative Examples 1 and 2) | | | | | | | | |
|---|---|---|---|---|---|---|---|---|
| Constituent component | | Example 1 | Example 2 | Example 3 | Comp. Example 1 | Example 4 | Comp. Example 2 | Example 5 |
| Base material | Ion-exchanged water | 49.72 | 50.22 | 49.22 | 48.42 | 48.72 | 48.22 | 49.22 |
| Component (a) | Iron oxide (red) with 300 nm mean particle size, surface-treated with silicone | 0.37 | 0.37 | 0.37 | 0.37 | 0.37 | 0.37 | 0.37 |
| | Iron oxide (yellow) with 250 nm mean particle size, surface-treated with silicone | 1.07 | 1.07 | 1.07 | 1.07 | 1.07 | 1.07 | 1.07 |
| | Iron oxide (black) with 300 nm mean particle size, surface-treated with silicone | 0.04 | 0.04 | 0.04 | 0.04 | 0.04 | 0.04 | 0.04 |
| | Titanium oxide with 250 nm mean particle size, surface-treated with silicone | 8.7 | 8.7 | 8.7 | 8.7 | 8.7 | 8.7 | 8.7 |
| Component (b) | Stearoxyhydroxypropyl methylcellulose | 0.2 | 0.2 | 0.2 | - | 0.2 | 0.2 | 0.2 |
| Nonionic surfactant with HLB value of ≥10 | PEG-60 hydrogenated castor oil | - | - | - | 1.5 | 1 | 1.5 | 0.5 |
| Component (c) | Polyhydroxystearic acid | 1.0 | 0.5 | 1.5 | 1.0 | 1.0 | 1.0 | 1.0 |
| Component (d) | Ethylhexyl methoxycinnamate (polar oil) | 7.5 | 7.5 | 7.5 | 7.5 | 7.5 | 7.5 | 7.5 |
| | Polypropylene glycol (polar oil) | 1.0 | 1.0 | 1.0 | 1.0 | 1.0 | 1.0 | 1.0 |
| | Diisopropyl sebacate (polar oil) | 4.0 | 4.0 | 4.0 | 4.0 | 4.0 | 4.0 | 4.0 |
| | Dimethicone(silicone oil) | - | - | - | - | - | - | - |
| Other components | Glycerin | 2.0 | 2.0 | 2.0 | 2.0 | 2.0 | 2.0 | 2.0 |
| | Tocopherol | 10 | 10 | 10 | 10 | 10 | 10 | 10 |
| | (Dimethylacrylamide/acryloyldimethyltaurine Na) cross polymer | 0.3 | 0.3 | 0.3 | 0.3 | 0.3 | 0.3 | 0.3 |
| | Ba sulfate | 1.0 | 1.0 | 1.0 | 1.0 | 1.0 | 1.0 | 1.0 |
| | HDI/trimethylolhexyllactone cross polymer | 1.0 | 1.0 | 1.0 | 1.0 | 1.0 | 1.0 | 1.0 |
| | Silica | 0.1 | 0.1 | 0.1 | 0.1 | 0.1 | 0.1 | 0.1 |
| | Ethanol | 12 | 12 | 12 | 12 | 12 | 12 | 12 |
| Total | | 100 | 100 | 100 | 100 | 100 | 100 | 100 |
| Emulsified particle size (µm) | | 1 to 10(20) | 1 to 10(20) | 1 to 10(20) | 1 to 5(20) | 1 to 5(20) | 1 to 3(15) | 1 to 5(20) |
| Evaluation results | Fresh sensation | A | A | B | B | B | B | B |
| | Covering power | A | A | A | A | A | A | A |
| | Emulsified stability (low-speed stirring test) | A | A | B | C | B | C | B |

**[Table 2]**

| Table 2 (Examples 6-8, Comparative Examples 3 and 4) | | | | | | |
|---|---|---|---|---|---|---|
| Constituent component | | Example 6 | Example 7 | Example 8 | Comp. Example 3 | Comp. Example 4 |
| Base material | Ion-exchanged water | 44.64 | 49.72 | 49.72 | 39.54 | 46.22 |
| Component (a) | Iron oxide (red) with 300 nm mean particle size, surface-treated with silicone | 0.55 | 0.37 | 0.37 | 0.74 | 0.37 |
| | Iron oxide (yellow) with 250 nm mean particle size, surface-treated with silicone | 1.6 | 1.07 | 1.07 | 2.14 | 1.07 |
| | Iron oxide (black) with 300 nm mean particle size, surface-treated with silicone | 0.06 | 0.04 | 0.04 | 0.08 | 0.04 |
| | Titanium oxide with 250 nm mean particle size, surface-treated with silicone | 13.05 | 8.7 | 8.7 | 17.4 | 8.7 |
| Component (b) | Stearoxyhydroxypropyl methylcellulose | 0.2 | 0.2 | 0.2 | 0.2 | 0.2 |
| Nonionic surfactant with HLB value of ≥10 | PEG-60 hydrogenated castor oil | - | - | - | - | - |
| Component (c) | Polyhydroxystearic acid | 1.0 | 1.0 | 1.0 | 1.0 | 1.0 |
| Component (d) | Ethylhexyl methoxycinnamate (polar oil) | 7.5 | 4.0 | 3.5 | 7.5 | - |
| | Polypropylene glycol (polar oil) | 1.0 | 0.5 | 0.4 | 1.0 | - |
| | Diisopropyl sebacate (polar oil) | 4.0 | 2.3 | 1.7 | 4.0 | - |
| | Dimethicone(1.5cs) (silicone oil) | - | - | - | - | 16 |
| | Isododecane (hydrocarbon oil) | - | 5.7 | 6.9 | - | - |
| Other components | Glycerin | 2.0 | 2.0 | 2.0 | 2.0 | 2.0 |
| | Tocopherol | 10 | 10 | 10 | 10 | 10 |
| | (Dimethylacrylamide/acryloyldimethyltaurine Na) cross polymer | 0.3 | 0.3 | 0.3 | 0.3 | 0.3 |
| | Ba sulfate | 1.0 | 1.0 | 1.0 | 1.0 | 1.0 |
| | HDI/trimethylolhexyllactone cross polymer | 1.0 | 1.0 | 1.0 | 1.0 | 1.0 |
| | Silica | 0.1 | 0.1 | 0.1 | 0.1 | 0.1 |
| | Ethanol | 12 | 12 | 12 | 12 | 12 |
| Total | | 100 | 100 | 100 | 100 | 100 |
| Emulsified particle size (µm) | | 1 to 10(20) | 1 to 10(20) | 1 to 10(20) | 1 to 10(20) | -* |
| Evaluation results | Fresh sensation | B | A | A | B | |
| | Covering power | A | A | A | A | |
| | Emulsified stability (low-speed stirring test) | B | A | B | C | |

| | | | | | | |
|---|---|---|---|---|---|---|
| Note*: No emulsification into a cosmetic composition, and therefore no measurement. | | | | | | |

As clearly shown in Table 1 and Table 2, the compositions of Examples 1 to 8 all had a characteristic fresh feel during use while exhibiting excellent emulsified stability and high covering power. In particular, superior results were obtained with the compositions of Examples 1, 2 and 7, for all of the evaluations.

In contrast, with the compositions of Comparative Examples 1 to 3 the covering power was high but the fresh sensation was relatively inferior, while the emulsified stability was poor. The composition of Comparative Example 4 which did not contain a polar oil failed to emulsify and could not serve as a cosmetic composition, and it was therefore unmeasurable.

As clearly seen by comparing Example 7 and Example 8, superior results were obtained with Example 7 in which 50 mass% or greater of the oil consisted of polar oil.

## Claims

1. An oil-in-water emulsified cosmetic composition comprising the following components (a) to (d):
(a) pigment particles with a mean particle size of 200 to 500 nm and hydrophobically treated on a surface,
(b) a water-soluble alkyl-substituted polysaccharide derivative,
(c) polyhydroxystearic acid, and
(d) an oil containing a polar oil,
wherein the pigment particles are dispersed in the oil,
wherein a content of the pigment particles is 8.0 mass% to 15 mass%, and
wherein a content of nonionic surfactants with an HLB value of 10 or greater is 1.0 mass% or lower.

2. The composition according to claim 1, wherein a content of the polyhydroxystearic acid is 0.1 mass% to 1.5 mass%.

3. The composition according to claim 1, wherein 50 mass% or greater of the oil is the polar oil.

4. The composition according to claim 1, wherein the water-soluble alkyl-substituted polysaccharide derivative is hydrophobic-modified hydroxypropyl methylcellulose.

5. The composition according to claim 1, wherein a content of the water-soluble alkyl-substituted polysaccharide derivative is 0.05 mass% to 1.0 mass%.

6. The composition according to claim 1, which further comprises an aqueous phase thickener.

7. The composition according to claim 1, wherein a content of the oil is 5.0 mass% to 40 mass%.

8. The composition according to claim 1, wherein the polar oil comprises an ultraviolet absorber.

9. The composition according to any one of claims 1 to 8, which is a makeup cosmetic composition.

10. The composition according to any one of claims 1 to 8, which is a foundation.
